# EUROPEAN PATENT SPECIFICATION

(11) **EP 4 043 058 B1**
(45) Date of publication and mention of the grant of the patent: **28.02.2024**
(21) Application number: 22155612.9
(22) Date of filing: 08.02.2022
(51) Int. Cl.: A61M 16/08, A61B 5/097

(54) **AIRWAY ADAPTER**
LUFTWEGEADAPTER
ADAPTATEUR POUR VOIES D'AÉRATION

(30) Priority: 15.02.2021 JP 2021021903
(43) Date of publication of application: 17.08.2022
(73) Proprietor: Nihon Kohden Corporation, Shinjuku-ku Tokyo (JP)
(72) Inventor: Takatori, Fumihiko, Tokorozawa-shi, Saitama (JP); Baba, Yuya, Tokorozawa-shi, Saitama (JP); Kabumoto, Kenichiro, Tokorozawa-shi, Saitama (JP); Aoyagi, Takayuki, Tokorozawa-shi, Saitama (JP); Suzuki, Kentaro, Tokorozawa-shi, Saitama (JP)
(74) Representative: Grünecker Patent- und Rechtsanwälte PartG mbB

(56) References cited:
- EP-A2- 0 054 714
- WO-A1-02/085207
- US-A1- 2010 036 272
- US-A1- 2010 083 969
- US-A1- 2011 028 858
- US-A1- 2011 284 000
- US-A1- 2016 184 545

## Description

### TECHNICAL FIELD

The present invention relates to an airway adapter which forms a part of a breathing circuit connected to a test subject.

### BACKGROUND ART

WO 2002/085207 discloses an airway adapter which forms a part of a breathing circuit connected to a test subject. The airway adapter is provided with a first cylindrical portion and a second cylindrical portion. The first cylindrical portion forms a male terminal that is fitted into a first member forming a part of the breathing circuit. The second cylindrical portion forms a female terminal that compartmentalizes a fitting portion into which a second member forming a part of the breathing circuit is fitted. The airway adapter is provided with an airway through which a space compartmentalized by the first cylindrical portion and the fitting portion communicate with each other.

EP 0 054 714 A2 discloses a connection system for fluid conduits with pluggable connection elements for a breathing or anesthetic apparatus. US 2010/0036272 A1 teaches a metabolic measurement system that includes an integrated airway adapter for monitoring breath-by-breath amounts of substances and anesthetic agents in the respiration of an individual.

### SUMMARY

An object of the present invention is to enhance user-friendliness of an airway adapter which may be used for a test subject whose ventilation volume is small.

According to an aspect in order to achieve the foregoing object, there is provided an airway adapter forming a part of a breathing circuit connected to a test subject, the airway adapter including:
a first cylindrical portion that forms a male terminal fitted into a female member forming another part of the breathing circuit;
a second cylindrical portion that forms a female terminal compartmentalizing a fitting portion into which a male member forming another part of the breathing circuit is fitted;
an airway forming portion that is disposed in an internal space compartmentalized by the first cylindrical portion and that forms an airway communicating with the fitting portion; and
a first sealing member that is disposed on an outer circumferential surface of the first cylindrical portion and that has higher flexibility than the first cylindrical portion.

### BRIEF DESCRIPTION OF DRAWINGS

FIG. 1 is an illustration of external appearance of an airway adapter according to an embodiment viewed from upper left front.
FIG. 2 is an illustration of the external appearance of the airway adapter in FIG. 1 viewed from upper right back.
FIG. 3 is an illustration of the external appearance of the airway adapter in FIG. 1 viewed from the front.
FIG. 4 is an illustration of the external appearance of the airway adapter in FIG. 1 viewed from the back.
FIG. 5 is an illustration of the external appearance of the airway adapter in FIG. 1 viewed from the left.
FIG. 6 is an illustration of the external appearance of the airway adapter in FIG. 1 viewed from above.
FIG. 7 is an illustration of a section taken along a line VII-VII in FIG. 3 and viewed from an arrow direction.
FIG. 8 is an illustration of external appearance of first sealing members of the airway adapter in FIG. 1.
FIG. 9 is an illustration of a comparative example in order to explain the shapes of the first sealing members.
FIG. 10 is a view of another example of the shapes of the first sealing members.
FIG. 11 is a view of another example of the shapes of the first sealing members.
FIG. 12 is a view of another example of the shapes of the first sealing members.
FIG. 13 is a view of another example of the shapes of the first sealing members.
FIG. 14 is a view of another example of the shape of a first cylindrical portion of the airway adapter in FIG. 1.
FIG. 15 is a view of another example of the shapes of the first sealing members.
FIG. 16 is a view of another example of the shape of a female member into which the first cylindrical portion is fitted.
FIG. 17 is a view of another example of the shapes of the first sealing members.
FIG. 18 is a view of another example of the shape of a second cylindrical portion of the airway adapter in FIG. 1.
FIG. 19 is a view of another example of the shapes of second sealing members.
FIG. 20 is a view of another example of the shape of a male member that is fitted into the second cylindrical portion.
FIG. 21 is a view of another example of the shapes of the second sealing members.
FIG. 22 is an illustration of an optical sensor that is mounted on the airway adapter in FIG. 1.

### DESCRIPTION OF EMBODIMENT

An embodiment will be described by way of example below in detail with reference to the accompanying drawings. In the accompanying drawings, an arrow F indicates a frontward direction of an illustrated structure. An arrow B indicates a backward direction of the illustrated structure. An arrow U indicates an upward direction of the illustrated structure. An arrow D indicates a downward direction of the illustrated structure. An arrow L indicates a leftward direction of the illustrated structure. An arrow R indicates a rightward direction of the illustrated structure. These directions are indicated for convenience of explanation and not intended to limit the posture of the illustrated structure in actual use.

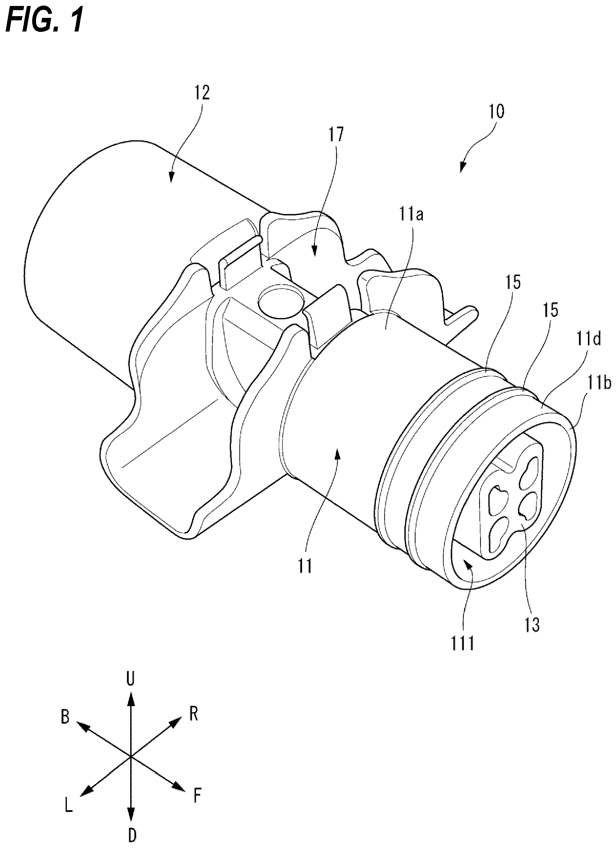

FIGS. 1 to 6 illustrate external appearance of an airway adapter 10 according to the embodiment. The airway adapter 10 is a medical device for forming a part of a breathing circuit connected to a test subject. The airway adapter 10 has a first cylindrical portion 11 and a second cylindrical portion 12.

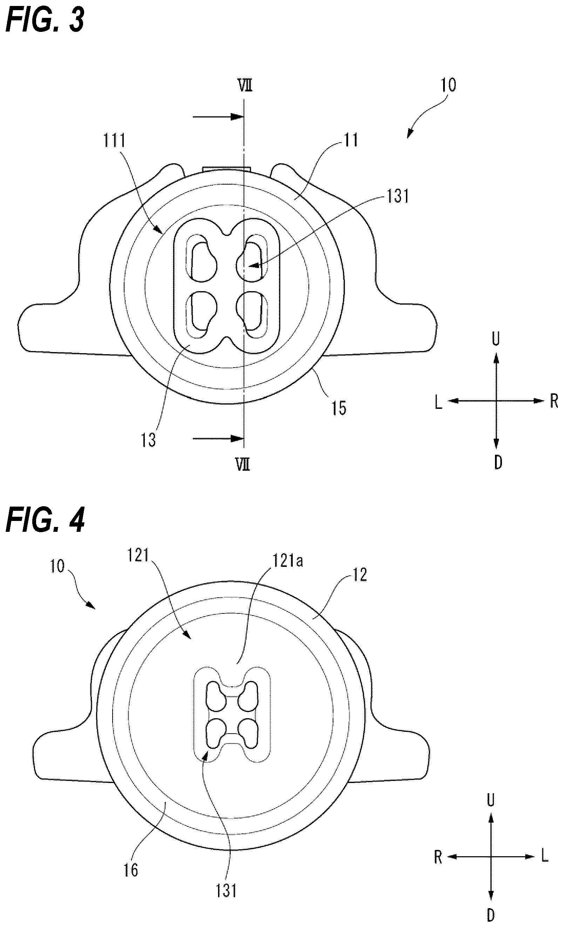

As illustrated in FIG. 1 and FIG. 3, the first cylindrical portion 11 compartmentalizes a bottomed internal space 111 on its radial-direction inner side. The first cylindrical portion 11 has a first proximal end portion 11a and a first distal end portion 11b. The first proximal end portion 11a is an end portion located nearer to the second cylindrical portion 12. The first distal end portion 11b is an end portion located farther from the second cylindrical portion 12. The internal space 111 is opened at the first distal end portion 11b.

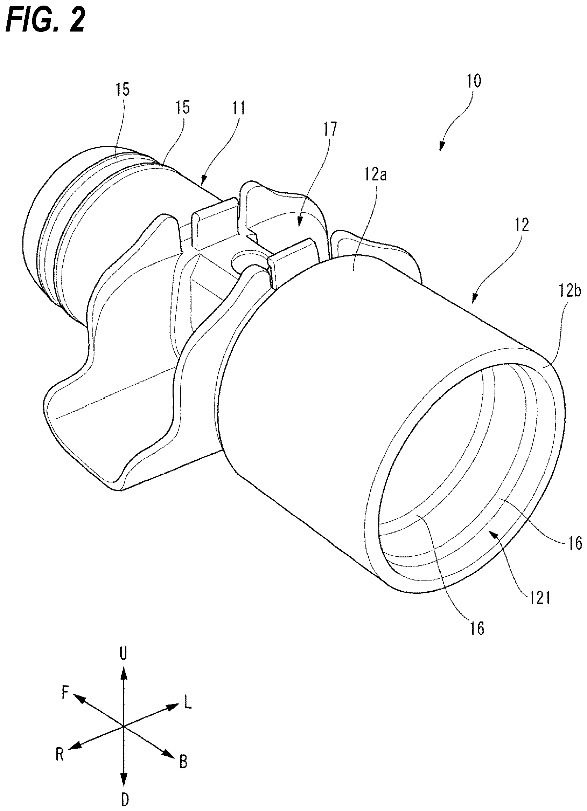

As illustrated in FIG. 2 and FIG. 4, the second cylindrical portion 12 compartmentalizes a fitting portion 121 on its radial-direction inner side. The second cylindrical portion 12 has a second proximal end portion 12a and a second distal end portion 12b. The second proximal end portion 12a is an end portion located nearer to the first cylindrical portion 11. The second distal end portion 12b is an end portion located farther from the first cylindrical portion 11. The fitting portion 121 that is a bottomed internal space is opened at the second distal end portion 12b.

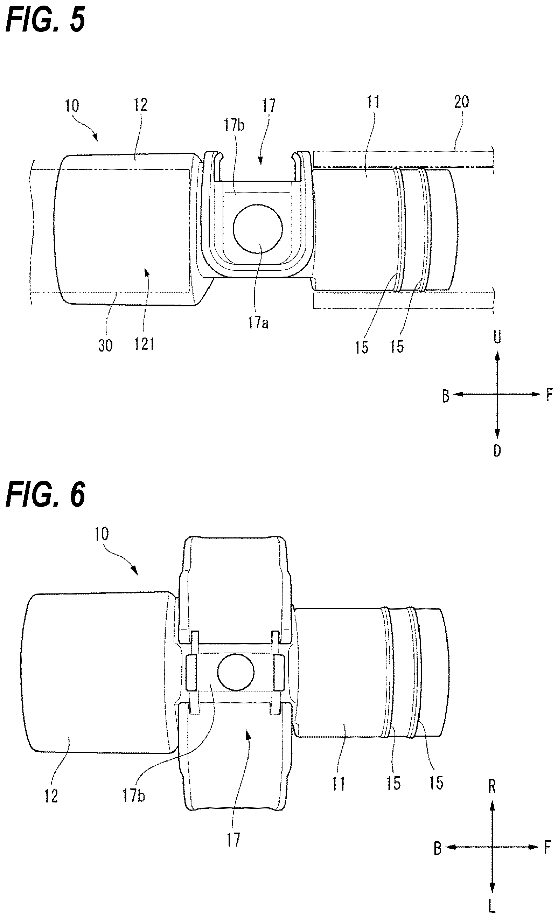

As illustrated in FIG. 5, the first cylindrical portion 11 forms a male terminal into which a female member 20 forming another part of the breathing circuit is fitted. The second cylindrical portion 12 forms a female terminal so that a male member 30 forming another part of the breathing circuit can be fitted into the fitting portion 121.

As illustrated in FIG. 1 and FIG. 3, the airway adapter 10 is provided with an airway forming portion 13. The airway forming portion 13 is disposed in the internal space 111 compartmentalized by the first cylindrical portion 11.

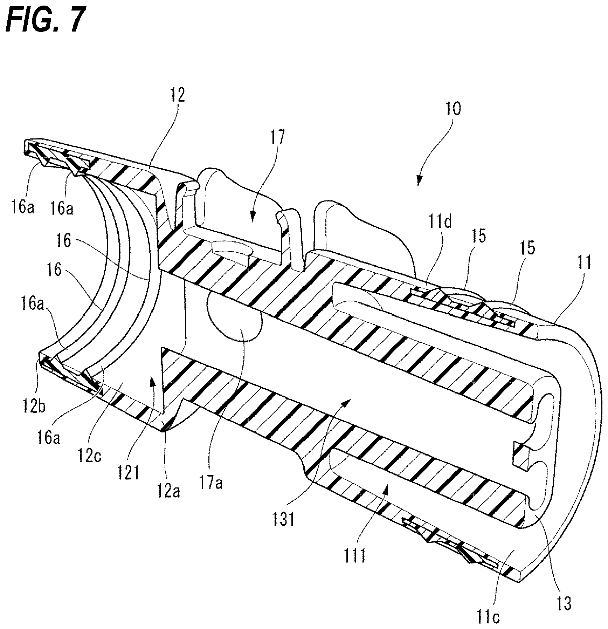

FIG. 7 illustrates a section of the airway adapter 10 taken along a line VII -VII in FIG. 3 and viewed from an arrow direction. The airway forming portion 13 extends along a direction in which the first cylindrical portion 11 is fitted into the female member 20 from a bottom portion 111a of the internal space 111 toward the opening. The airway forming portion 13 forms an airway 131.

As illustrated in FIG. 4 and FIG. 7, the airway 131 is opened at a bottom portion 121a of the fitting portion 121 that is compartmentalized by the second cylindrical portion 12. Thus, through the airway 131, the internal space 111 and the fitting portion 121 communicate with each other.

As illustrated in FIG. 1, FIG. 3, and FIGS. 5 to 7, the airway adapter 10 is provided with first sealing members 15. The first sealing members 15 are disposed on an outer circumferential surface 11d of the first cylindrical portion 11. The first sealing members 15 have higher flexibility than the first cylindrical portion 11. For example, the first cylindrical portion 11 may be molded from a resin such as polyethylene, polypropylene or polyethylene terephthalate. On the other hand, the first sealing members 15 may be molded from a urethane-based elastomer, silicon, or the like.

As illustrated in FIG. 5, when the first cylindrical portion 11 is fitted into the female member 20, the first sealing members 15 abut against an inner circumferential surface of the female member 20. Thus, airtightness and watertightness between the first cylindrical portion 11 and the female member 20 are secured.

By the configuration according to the present embodiment, the first sealing members 15 having the higher flexibility than the first cylindrical portion 11 are provided on the outer circumferential surface 11d of the first cylindrical portion 11. Accordingly, stress generated between the first cylindrical portion 11 and the female member 20 when the first cylindrical portion 11 is fitted into the female member 20 can be absorbed by deformation of the first sealing members 15. Thus, it is possible to secure airtightness and watertightness between the first cylindrical portion 11 and the female member 20 while suppressing resistance generated when the first cylindrical portion 11 is fitted into the female member 20. In addition, the resistance generated during the fitting can be suppressed, thereby easily securing a large fitting depth with which the first cylindrical portion 11 is fitted into the female member 20. Thus, a volume of a dead space that can be created in the breathing circuit can be reduced by use of the airway adapter 10. This fact is advantageous to a test subject whose ventilation volume is small enough to increase an effect of the dead space relatively. Consequently, it is possible to enhanced user-friendliness of the airway adapter 10, which may be used especially on the test subject whose ventilation volume is small.

As illustrated in FIG. 1, the first sealing members 15 are disposed at positions nearer to the first distal end portion 11b of the first cylindrical portion 11 than to the first proximal end portion 11a of the first cylindrical portion 11.

By such a configuration, it is possible to reduce dependence of the airtightness and watertightness secured by the first sealing members 15 on the fitting depth with which the first cylindrical portion 11 is fitted into the female member 20.

Although not shown, the first sealing members 15 may be alternatively disposed at positions nearer to the first proximal end portion 11a of the first cylindrical portion 11 than to the first distal end portion 1 1b of the first cylindrical portion 11. In this case, resistance generated when the first cylindrical portion 11 is fitted into the female member 20 can be reduced.

In the present embodiment, the two first sealing members 15 are arrayed along the direction in which the first cylindrical portion 11 is fitted into the female member 20. By such a configuration, it is possible to enhance strength and durability of the secured airtightness and watertightness.

The number of the first sealing members 15 may be suitably determined according to required airtightness and watertightness or required fitting resistance. A single first sealing member 15 may be provided alternatively, or three or more first sealing members 15 may be provided alternatively.

In the present embodiment, each of the first sealing members 15 has a ring shape that extends along the circumferential direction of the first cylindrical portion 11. In other words, the first sealing member 15 extends continuously without interruption along the circumferential direction of the first cylindrical portion 11. Also by such a configuration, it is possible to enhance the strength and durability of the secured airtightness and watertightness.

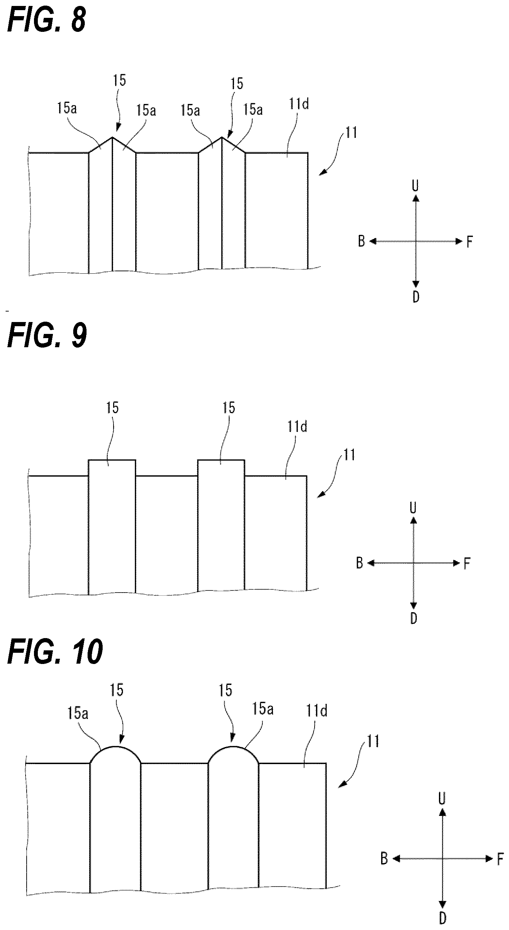

As illustrated in FIG. 8, the first sealing member 15 protrudes in the radial direction of the first cylindrical portion 11 from the outer circumferential surface 11d of the first cylindrical portion 11. In addition, the first sealing member 15 has inclined surfaces 15a each of which is inclined along the direction in which the first cylindrical portion 11 is fitted into the female member 20. Also by such a configuration, it is possible to reduce the resistance generated when the first cylindrical portion 11 is fitted into the female member 20.

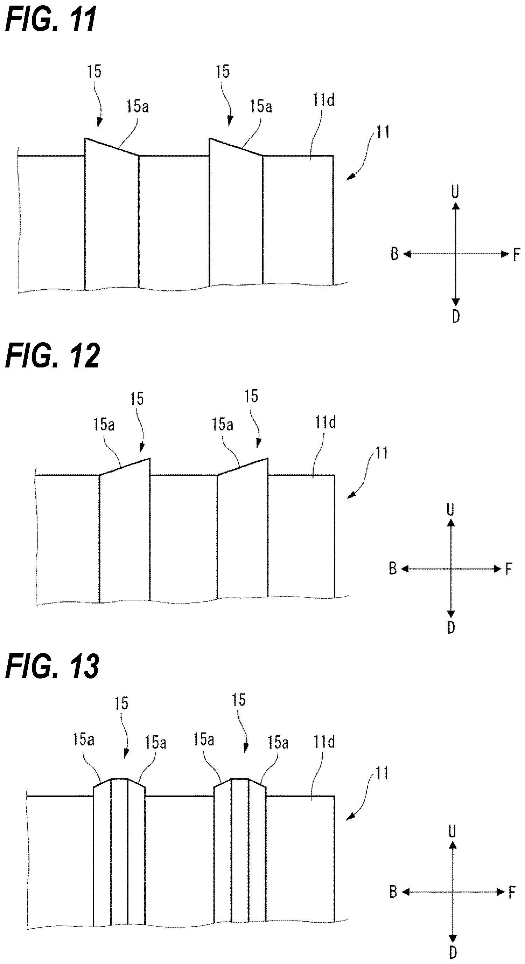

In the present example, the inclined surfaces 15a are formed so that the first sealing member 15 presents a triangular shape when viewed from the left/right direction of the airway adapter 10. The shapes of the inclined surfaces 15a may be determined suitably as long as a bottom portion and a top portion together with opposite end portions of the first sealing member 15 are not connected by vertical walls in the directions in which the first cylindrical portion 11 is inserted into and removed from the female member 20, as illustrated in FIG. 9. The shapes of the inclined surfaces 15a may be determined so that each of the first sealing members 15 presents a trapezoidal shape or a semicircular shape, as illustrated in any of FIGS. 10 to 13, when viewed from the left/right direction of the airway adapter 10.

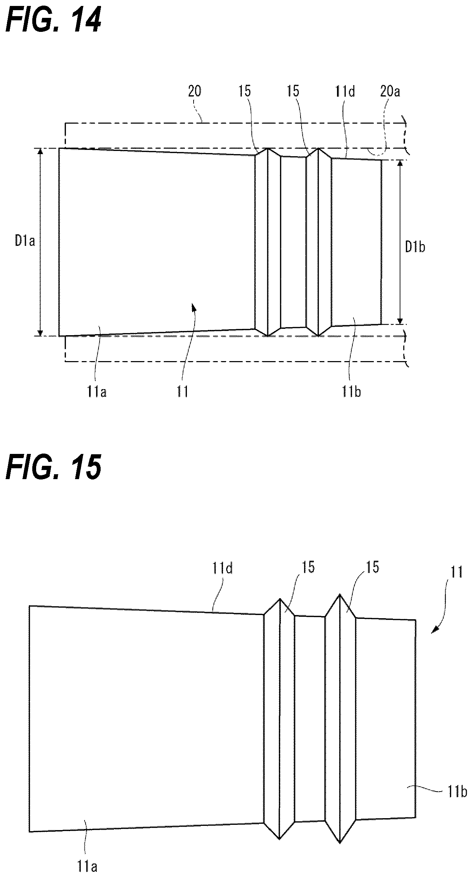

The first cylindrical portion 11 may be configured to have such a dimension in the radial direction that a value D1b at the first distal end portion 11b is smaller than a value D1a at the first proximal end portion 11a, as illustrated in FIG. 14.

By such a configuration, it is possible to more easily secure a large fitting depth with which the first cylindrical portion 11 is fitted into the female member 20. As a result, a gap is apt to be generated between the inner circumferential surface 20a of the female member 20 and the outer circumferential surface 11d of the first cylindrical portion 11. Due to the gap sealed by the first sealing members 15, degradation of the airtightness and watertightness can be suppressed. Therefore, a volume of a dead space that may be created in the breathing circuit can be reduced by use of the airway adapter 10 while the degradation of the airtightness and watertightness between the first cylindrical portion 11 and the female member 20 is suppressed.

The two first sealing members 15 have different protrusion heights from the outer circumferential surface 11d of the first cylindrical portion 11, as illustrated in FIG. 15. Specifically, the two first sealing members 15 are configured so that the first sealing member 15 provided at a position nearer to the first distal end portion 11b of the first cylindrical portion 11 is larger in protrusion height than the first sealing member 15 provided at a position nearer to the first proximal end portion 11a of the first cylindrical portion 11. As can be seen from comparison with FIG. 14, the first sealing member 15 having the larger protrusion height is disposed at the position where an interval between the inner circumferential surface 20a of the female member 20 and the outer circumferential surface 11d of the first cylindrical portion 11 is larger when the first cylindrical portion 11 is fitted into the female member 20.

By such a configuration, it is possible to further suppress the degradation of the airtightness and watertightness between the first cylindrical portion 11 and the female member 20 while easily securing the large fitting depth with which the first cylindrical portion 11 is fitted into the female member 20.

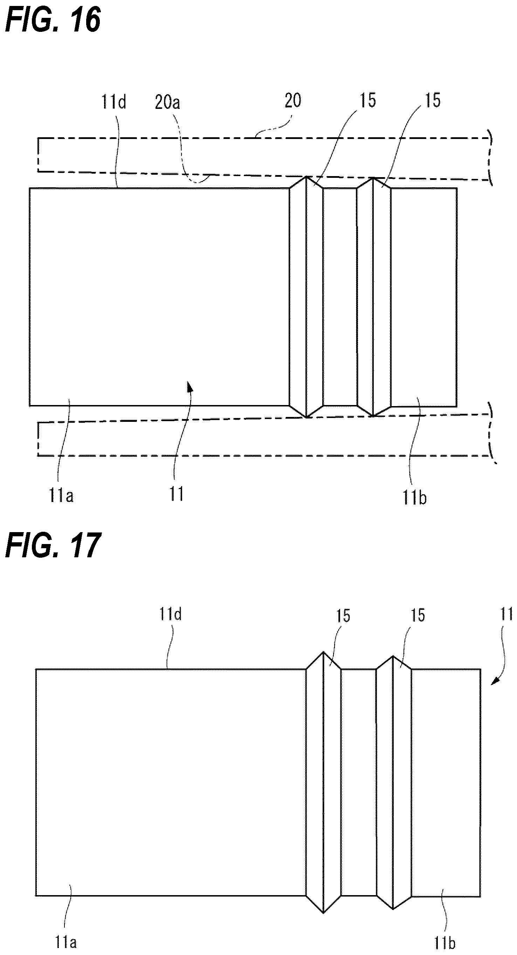

Incidentally, also by a configuration in which the first cylindrical portion 11 whose outer diameter is constant is fitted into the female member 20 whose inner diameter gradually decreases, as illustrated in FIG. 16, it is possible to easily secure a large fitting depth with which the first cylindrical portion 11 is fitted into the female member 20.

In this case, first sealing members 15 may be configured so that the first sealing member 15 provided at a position nearer to the first proximal end portion 11a is larger in protrusion height than the first sealing member 15 provided at a position nearer to the first distal end portion 11b of the first cylindrical portion 11, as illustrated in FIG. 17. As can be seen from comparison with FIG. 16, the first sealing member 15 having the larger protrusion height is disposed at the position where an interval between the inner circumferential surface 20a of the female member 20 and the outer circumferential surface 11d of the first cylindrical portion 11 is larger when the first cylindrical portion 11 is fitted into the female member 20.

Also by such a configuration, it is possible to further suppress the degradation of the airtightness and watertightness between the first cylindrical portion 11 and the female member 20 while easily securing the large fitting depth with which the first cylindrical portion 11 is fitted into the female member 20.

Incidentally, between the first cylindrical portion 11 whose outer diameter gradually decreases toward the first distal end portion 11b and the female member 20 whose inner diameter gradually decreases from the distal end, it is possible to easily secure the large fitting depth with which the first cylindrical portion 11 is fitted into the female member 20.

As illustrated in FIG. 2, FIG. 4 and FIG. 7, the airway adapter 10 may be provided with second sealing members 16. The second sealing members 16 are disposed in a fitting portion 121 that is compartmentalized by the second cylindrical portion 12. The second sealing members 16 have higher flexibility than the second cylindrical portion 12. For example, the second cylindrical portion 12 may be molded from a resin such as polyethylene, polypropylene, or polyethylene terephthalate. On the other hand, the second sealing members 16 may be molded from a urethane-based elastomer, silicon, or the like.

Although not shown, the second sealing members 16 abut against an outer circumferential surface of the male member 30 when the male member 30 is fitted into the fitting portion 121, as illustrated in FIG. 5. Due to the second sealing members 16 having the higher flexibility than the second cylindrical portion 12, stress generated between the male member 30 and the fitting portion 121 when the male member 30 is fitted into the fitting portion 121 can be absorbed by deformation of the second sealing members 16. Thus, it is possible to secure airtightness and watertightness also between the second cylindrical portion 12 and the male member 30 while suppressing resistance generated when the male member 30 is fitted into the fitting portion 121. In addition, the resistance generated during the fitting can be suppressed, thereby easily securing a large fitting depth with which the male member 30 is fitted into the fitting portion 121. Thus, a volume of a dead space that can be created in the breathing circuit can be reduced by use of the airway adapter 10. This fact is advantageous to a test subject whose ventilation volume is small enough to increase an effect of the dead space relatively.

As illustrated in FIG. 7, the second sealing members 16 are disposed at positions nearer to the second distal end portion 12b of the second cylindrical portion 12 than to the second proximal end portion 12a of the second cylindrical portion 12.

By such a configuration, it is possible to reduce dependence of the airtightness and watertightness secured by the second sealing members 16 on the fitting depth with which the male member 30 is fitted into the second cylindrical portion 12.

Although not shown, the second sealing members 16 may be alternatively disposed at positions nearer to the second proximal end portion 12a of the second cylindrical portion 12 than to the second distal end portion 12b of the second cylindrical portion 12. In this case, it is possible to reduce resistance generated when the male member 30 is fitted into the fitting portion 121.

In the present embodiment, the two second sealing members 16 are arrayed along the direction in which the male member 30 is fitted into the fitting portion 121. By such a configuration, it is possible to enhance strength and durability of the secured airtightness and watertightness.

The number of the second sealing members 16 may be determined suitably according to required airtightness and watertightness or required fitting resistance. A single second sealing member 16 may be provided alternatively, or three or more second sealing members 16 may be provided alternatively.

In the present embodiment, each of the second sealing members 16 has a ring shape that extends along the circumferential direction of the second cylindrical portion 12. That is, the second sealing member 16 extends continuously without interruption along the circumferential direction of the second cylindrical portion 12. Also by such a configuration, it is possible to enhance strength and durability of the secured airtightness and watertightness.

As illustrated in FIG. 7, the second sealing member 16 protrudes in the radial direction of the second cylindrical portion 12 from an inner circumferential surface 12c of the second cylindrical portion 12. In addition, the second sealing member 16 has inclined surfaces 16a each of which is inclined along the direction in which the male member 30 is fitted into the fitting portion 121. Also by such a configuration, it is possible to reduce the resistance generated when the male member 30 is fitted into the fitting portion 121.

In the present example, the inclined surfaces 16a are formed so that the second sealing member 16 presents a triangular shape when viewed from the left/right direction of the airway adapter 10. The shapes of the inclined surfaces 16a may be determined suitably like any of the first sealing members 15 that have been described with reference to FIGS. 10 to 13, as long as a bottom portion and a top portion of the second sealing member 16 are not connected by vertical walls in the directions in which the male member 30 is inserted into and removed from the fitting portion 121, like the first sealing member 15 that has been described with reference to FIG. 9. For example, the shapes of the inclined surfaces 16a may be determined so that the second sealing member 16 presents a trapezoidal or semicircular shape when viewed from the right/left direction of the airway adapter 10.

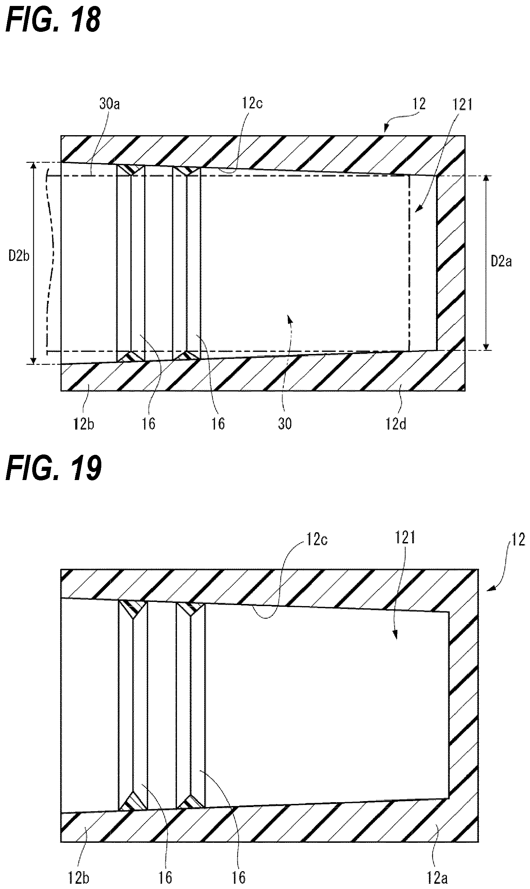

The fitting portion 121 compartmentalized by the second cylindrical portion 12 may be configured to have such a dimension in the radial direction that a value D2a at the second proximal end portion 12a is smaller than a value D2b at the second distal end portion 12b, as illustrated in FIG. 18.

By such a configuration, it is possible to more easily secure a large fitting depth with which the male member 30 is fitted into the fitting portion 121. As a result, a gap is apt to be generated between the inner circumferential surface 12c of the second cylindrical portion 12 and the outer circumferential surface 30a of the male member 30. Since the gap is sealed by the second sealing members 16, degradation of airtightness and watertightness can be suppressed. Accordingly, a volume of a dead space that may be generated in the breathing circuit can be reduced by use of the airway adapter 10 while the degradation of the airtightness and watertightness between the second cylindrical portion 12 and the male member 30 is suppressed.

The two second sealing members 16 have different protrusion heights from the inner circumferential surface 12c of the second cylindrical portion 12, as illustrated in FIG. 19. Specifically, the two second sealing members 16 are configured so that the second sealing member 16 provided at a position nearer to the second distal end portion 12b of the second cylindrical portion 12 is larger in protrusion height than the second sealing member 16 provided at a position nearer to the second base portion 12a of the second cylindrical portion 12. As can be seen from comparison with FIG. 18, the second sealing member 16 having the larger protrusion height is disposed at the position where an interval between the outer circumferential surface 30a of the male member 30 and the inner circumferential surface 12c of the second cylindrical portion 12 is larger when the male member 30 is fitted into the fitting portion 121.

By such a configuration, it is possible to further suppress the degradation of the airtightness and watertightness between the second cylindrical portion 12 and the male member 30 while easily securing the large fitting depth with which the male member 30 is fitted into the fitting portion 121.

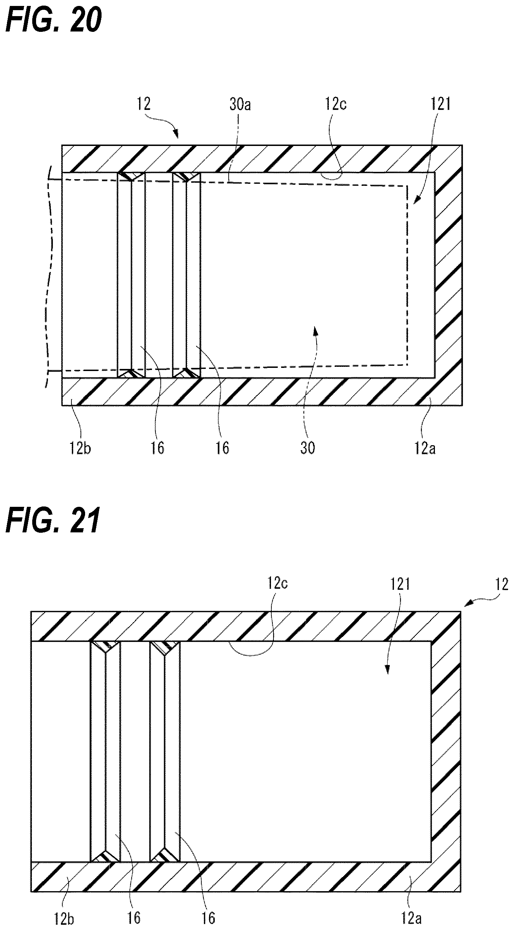

Incidentally, the large fitting depth with which the male member 30 is fitted into the fitting portion 121 can be easily secured also by a configuration in which the male member 30 whose outer diameter gradually decreases toward the distal end is fitted into the second cylindrical portion 12 in which the inner diameter of the fitting portion 121 is constant, as illustrated in FIG. 20.

In this case, the second sealing members 16 may be configured so that the second sealing member 16 provided at a position nearer to the second proximal end portion 12a of the second cylindrical portion 12 is larger in protrusion height than the second sealing member 16 provided at a position nearer to the second distal end portion 12b of the second cylindrical portion 12, as illustrated in FIG. 21. As can be seen from comparison with FIG. 20, the second sealing member 16 having the larger protrusion height is disposed at the position where an interval between the outer circumferential surface 30a of the male member 30 and the inner circumferential surface 12c of the second cylindrical portion 12 is larger when the male member 30 is fitted into the fitting portion 121.

Also by such a configuration, it is possible to further suppress the degradation of the airtightness and watertightness between the second cylindrical portion 12 and the male member 30 while easily securing the large fitting depth with which the male member 30 is fitted into the fitting portion 121.

Incidentally, also by the combination of the fitting portion 121 whose inner diameter gradually decreases toward the second proximal end portion 12a and the male member 30 whose outer diameter gradually decreases toward the distal end, the large fitting depth with which the male member 30 is fitted into the fitting portion 121 can be secured.

As illustrated in FIG. 1, FIG. 2, FIG. 5, and FIG. 6, the airway adapter 10 is provided with a sensor support portion 17. The sensor support portion 17 is disposed between the first cylindrical portion 11 and the second cylindrical portion 12. The sensor support portion 17 is provided with a convex portion 17b in which a pair of windows 17a are formed. As is apparent from FIG. 5 and FIG. 7, the pair of windows 17a face each other across the airway 131.

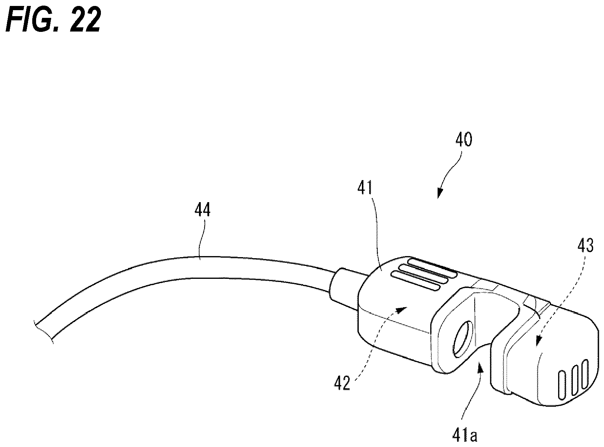

An optical sensor 40 that is illustrated in FIG. 22 may be mounted on the sensor support portion 17. The optical sensor 40 is provided with a housing 41. The housing 41 has a concave portion 41a. The housing 41 receives a light-emitting element 42 and a light-detecting element 43. The light-emitting element 42 and the light-detecting element 43 are disposed so as to face each other across the concave portion 41a.

The optical sensor 40 is provided with a lead wire 44. The lead wire 44 includes a signal line that transmits a drive signal from a not-shown measurement device to the light-emitting element 42. Light emitted from the light-emitting element 42 based on the drive signal passes through the concave portion 41a to be incident on the light-detecting element 43. The light-detecting element 43 outputs a detection signal corresponding to intensity of the incident light. The lead wire 44 also includes a signal line that transmits the detection signal to the measurement device.

When the optical sensor 40 is mounted on the sensor support portion 17, the convex portion 17b is fitted into the concave portion 41a. Thus, the optical sensor 40 is supported by the sensor support portion 17. On this occasion, the light emitted from the light-emitting element 42 enters the airway 131 through one of the paired windows 17a provided in the convex portion 17b. The light that has passed through the airway 131 is incident on the light-detecting element 43 through the other of the paired windows 17a.

The optical sensor 40 is used to identify concentration of a specific gas component contained in exhaled air from a test subject. The wavelength of the light emitted from the light-emitting element 42 is defined as a wavelength that is significantly absorbed by the specific gas component. Examples of the specific gas component include carbon dioxide and anesthetic gas.

The exhaled air from the test subject is led to the airway 131 through the breathing circuit. The concentration of the specific gas component in the airway 131 located between the light-emitting element 42 and the light-detecting element 43 of the optical sensor 40 changes in accordance with the breathing of the test subject, so that intensity of the detected light in the light-detecting element 43 changes. In this manner, it is possible to measure the change over time of the concentration of the gas component in the breathing air of the test subject.

The aforementioned embodiment is merely exemplified to facilitate understanding of the present invention. The configuration according to the aforementioned embodiment may be changed/improved suitably without departing from the scope of the present invention as defined by the appended claims.

The first cylindrical portion 11 does not always need to be circularly cylindrical in shape. A first cylindrical portion 11 having a rectangularly cylindrical shape or the like may be alternatively used as long as the first cylindrical portion 11 can compartmentalize the internal space 111. In this case, the "radial direction of the first cylindrical portion 11" in the above description may be defined as a direction perpendicular to the direction in which the first cylindrical portion 11 is fitted into the female member 20.

Similarly, the second cylindrical portion 12 does not always need to be circularly cylindrical in shape. A second cylindrical portion 12 having a square cylindrical shape or the like may be alternatively used as long as the second cylindrical portion 12 can compartmentalize the fitting portion 121. In this case, the "radial direction of the second cylindrical portion 12" in the above description can be defined as a direction perpendicular to the direction in which the male member 30 is fitted into the fitting portion 121.

At least either the second sealing members 16 or the sensor support portion 17 may be omitted if necessary.

## Claims

1. An airway adapter (10) forming a part of a breathing circuit connected to a test subject, the airway adapter comprising:
a first cylindrical portion (11) that forms a male terminal fitted into a female member (20) forming another part of the breathing circuit;
a second cylindrical portion (12) that forms a female terminal compartmentalizing a fitting portion (121) into which a male member (30) forming another part of the breathing circuit is fitted;
an airway forming portion (13) that is disposed in an internal space (111) compartmentalized by the first cylindrical portion (11) and that forms an airway (131) communicating with the fitting portion (121); and
a first sealing member (15) that is disposed on an outer circumferential surface (11d) of the first cylindrical portion (11) and that has higher flexibility than the first cylindrical portion (11).

2. The airway adapter (10) according to Claim 1, wherein
the first sealing member (15) has a ring shape extending in a circumferential direction of the first cylindrical portion (11).

3. The airway adapter (10) according to Claim 1 or 2, wherein
the first cylindrical portion (11) has a first proximal end portion (11a) that is nearer to the second cylindrical portion (12), and a first distal end portion (11b) that is farther from the second cylindrical portion (12); and
the first sealing member (15) is disposed at a position nearer to the first distal end portion (11b) than to the first proximal end portion (11a).

4. The airway adapter (10) according to Claim 1 or 2, wherein
the first cylindrical portion (11) has a first proximal end portion (11a) that is nearer to the second cylindrical portion (12), and a first distal end portion (11b) that is farther from the second cylindrical portion (12); and
the first sealing member (15) is disposed at a position nearer to the first proximal end portion (11a) than to the first distal end portion (11b).

5. The airway adapter (10) according to any one of Claims 1 to 4, wherein
the first cylindrical portion (11) has a first proximal end portion (11a) that is nearer to the second cylindrical portion (12), and a first distal end portion (11b) that is farther from the second cylindrical portion (12); and
a dimension in a radial direction of the first cylindrical portion (11) is smaller in value at the first distal end portion (11b) than at the first proximal end portion (11a).

6. The airway adapter (10) according to any one of Claims 1 to 5, wherein
the first sealing member (15) includes a plurality of sealing members that are arrayed along a direction in which the male terminal is fitted into the female member (20).

7. The airway adapter (10) according to Claim 6, wherein
the first sealing members (15) consist of at least two first sealing members having different protrusion heights from the outer circumferential surface (11d); and
one of the first sealing members (15) that has a larger protrusion height is disposed at a position where an interval between an inner circumferential surface of the female member (20) and the outer circumferential surface (11d) is larger when the first cylindrical portion (11) is fitted into the female member (20).

8. The airway adapter (10) according to any one of Claims 1 to 7, wherein
the first sealing member (15) protrudes in a radial direction of the first cylindrical portion (11) from the outer circumferential surface (11d) of the first cylindrical portion (11), and has a surface inclined along a direction in which the male terminal is fitted into the female member (20).

9. The airway adapter (10) according to any one of Claims 1 to 8, further comprising:
a second sealing member (16) that is disposed on an inner circumferential surface (12c) of the second cylindrical portion (12) in the fitting portion (121), and that has higher flexibility than the second cylindrical portion (12).

10. The airway adapter (10) according to Claim 9, wherein
the second sealing member (16) has a ring shape that extends in a circumferential direction of the second cylindrical portion (12).

11. The airway adapter (10) according to Claim 9 or 10, wherein
the fitting portion (121) has a second proximal end portion (12a) that is nearer to the first cylindrical portion (11), and a second distal end portion (12b) that is farther from the first cylindrical portion (11); and
the second sealing member (16) is disposed at a position nearer to the second distal end portion (12b) than to the second proximal end portion (12a).

12. The airway adapter (10) according to Claim 9 or 10, wherein
the fitting portion (121) has a second proximal end portion (12a) that is nearer to the first cylindrical portion (11), and a second distal end portion (12b) that is farther from the first cylindrical portion (11); and
the second sealing member (16) is disposed at a position nearer to the second proximal end portion (12a) than to the second distal end portion (12b).

13. The airway adapter (10) according to any one of Claims 9 to 12, wherein
the second cylindrical portion (12) has a second proximal end portion (12a) that is nearer to the first cylindrical portion (11), and a second distal end portion (12b) that is farther from the first cylindrical portion (11); and
a dimension of the fitting portion (121) in the radial direction of the second cylindrical portion (12) is smaller in value at the second proximal end portion (12a) than at the second distal end portion (12b).

14. The airway adapter (10) according to any one of Claims 9 to 13, wherein
the second sealing member (16) includes a plurality of sealing members that are arrayed along a direction in which the male member (30) is fitted.

15. The airway adapter (10) according to Claim 14, wherein
the second sealing members (16) consist of at least two second sealing members having different protrusion heights from the inner circumferential surface (12c); and
one of the second sealing members (16) that has a larger protrusion height is disposed at a position where an interval between an outer circumferential surface of the male member (30) and the inner circumferential surface (12c) is larger when the male member (30) is fitted into the fitting portion (121).

16. The airway adapter (10) according to any one of Claims 9 to 15, wherein
the second sealing member (16) protrudes in a radial direction of the first cylindrical portion (11) from an inner circumferential surface (12c) of the second cylindrical portion (12) and has an inclined surface extending along a direction in which the male member (30) is fitted.

17. The airway adapter (10) according to any one of Claims 1 to 16, further comprising:
a support portion (17) that is disposed between the first cylindrical portion (11) and the second cylindrical portion (12), and on which a sensor (40) for detecting gas in the airway (131) is mounted.

## Patentansprüche

1. Atemwegadapter (10), der einen Teil eines Atemkreislaufs bildet, der mit einer Testperson verbunden ist, wobei der Atemwegadapter umfasst:
einen ersten zylindrischen Abschnitt (11), der einen Einsteckanschluss bildet, der in ein Aufnahmeelement (20) eingepasst wird, das einen anderen Teil des Atemkreislaufs bildet;
einen zweiten zylindrischen Abschnitt (12), der einen Aufnahmeanschluss bildet, der einen Passabschnitt (121) unterteilt, in den ein Einsteckelement (30) eingepasst wird, das einen anderen Teil des Atemkreislaufs bildet;
einen einen Atemweg bildenden Abschnitt (13), der in einem Innenraum (111) angeordnet ist, der durch den ersten zylindrischen Abschnitt (11) unterteilt wird, und der einen Atemweg (131) bildet, der mit dem Passabschnitt (121) in Verbindung steht; sowie
ein erstes Dichtungselement (15), das an einer Außenumfangsfläche (11d) des ersten zylindrischen Abschnitts (11) angeordnet ist und das höhere Flexibilität aufweist als der erste zylindrische Abschnitt (11).

2. Atemwegadapter (10) nach Anspruch 1, wobei
das erste Dichtungselement (15) eine Ringform hat, die sich in einer Umfangsrichtung des ersten zylindrischen Abschnitts (11) erstreckt.

3. Atemwegadapter (10) nach Anspruch 1 oder 2, wobei
der erste zylindrische Abschnitt (11) einen ersten proximalen Endabschnitt (11a), der näher an dem zweiten zylindrischen Abschnitt (12) liegt, sowie einen ersten distalen Endabschnitt (11b) aufweist, der weiter von dem zweiten zylindrischen Abschnitt (12) entfernt ist; und
das erste Dichtungselement (15) an einer Position angeordnet ist, die näher an dem ersten distalen Endabschnitt (11b) liegt als an dem ersten proximalen Endabschnitt (11a).

4. Atemwegadapter (10) nach Anspruch 1 oder 2, wobei
der erste zylindrische Abschnitt (11) einen ersten proximalen Endabschnitt (11a), der näher an dem zweiten zylindrischen Abschnitt (12) liegt, sowie einen ersten distalen Endabschnitt (11b) aufweist, der weiter von dem zweiten zylindrischen Abschnitt (12) entfernt ist; und
das erste Dichtungselement (15) an einer Position angeordnet ist, die näher an dem ersten proximalen Endabschnitt (11a) liegt als an dem ersten distalen Endabschnitt (11b).

5. Atemwegadapter (10) nach einem der Ansprüche 1 bis 4, wobei
der erste zylindrische Abschnitt (11) einen ersten proximalen Endabschnitt (11a), der näher an dem zweiten zylindrischen Abschnitt (12) liegt, sowie einen ersten distalen Endabschnitt (11b) aufweist, der weiter von dem zweiten zylindrischen Abschnitt (12) entfernt ist; und
eine Abmessung des ersten zylindrischen Abschnitts (11) in einer radialen Richtung an dem ersten distalen Endabschnitt (11b) einen kleineren Wert hat als an dem ersten proximalen Endabschnitt (11a).

6. Atemwegadapter (10) nach einem der Ansprüche 1 bis 5, wobei
das erste Dichtungselement (15) eine Vielzahl von Dichtungselementen einschließt, die in einer Richtung angeordnet sind, in der der Einsteckanschluss in das Aufnahmeelement (20) eingepasst ist.

7. Atemwegadapter (10) nach Anspruch 6, wobei
die ersten Dichtungselemente (15) aus wenigstens zwei ersten Dichtungselementen bestehen, die um unterschiedliche Höhen von der Außenumfangsfläche (11d) vorstehen; und
eines der ersten Dichtungselemente (15), das um eine größere Höhe vorsteht, an einer Position angeordnet ist, an der ein Abstand zwischen einer Innenumfangsfläche des Aufnahmeelementes (20) und der Außenumfangsfläche (11d) größer ist, wenn der erste zylindrische Abschnitt (11) in das Aufnahmeelement (20) eingepasst ist.

8. Atemwegadapter (10) nach einem der Ansprüche 1 bis 7, wobei
das erste Dichtungselement (15) in einer radialen Richtung des ersten zylindrischen Abschnitts (11) von der Außenumfangsfläche (11d) des ersten zylindrischen Abschnitts (11) vorsteht und eine Fläche hat, die in einer Richtung geneigt ist, in der der Einsteckanschluss in das Aufnahmeelement (20) eingepasst ist.

9. Atemwegadapter (10) nach einem der Ansprüche 1 bis 8, der des Weiteren umfasst:
ein zweites Dichtungselement (16), das an einer Innenumfangsfläche (12c) des zweiten zylindrischen Abschnitts (12) in dem Passabschnitt (121) angeordnet ist und das höhere Flexibilität aufweist als der zweite zylindrische Abschnitt (12).

10. Atemwegadapter (10) nach Anspruch 9, wobei
das zweite Dichtungselement (16) eine Ringform hat, die sich in einer Umfangsrichtung des zweiten zylindrischen Abschnitts (12) erstreckt.

11. Atemwegadapter (10) nach Anspruch 9 oder 10, wobei
der Passabschnitt (121) einen zweiten proximalen Endabschnitt (12a), der näher an dem ersten zylindrischen Abschnitt (11) liegt, sowie einen zweiten distalen Endabschnitt (12b) aufweist, der weiter von dem ersten zylindrischen Abschnitt (11) entfernt ist; und
das zweite Dichtungselement (16) an einer Position angeordnet ist, die näher an dem zweiten distalen Endabschnitt (12b) liegt als an dem zweiten proximalen Endabschnitt (12a).

12. Atemwegadapter (10) nach Anspruch 9 oder 10, wobei
der Passabschnitt (121) einen zweiten proximalen Endabschnitt (12a), der näher an dem ersten zylindrischen Abschnitt (11) liegt, sowie einen zweiten distalen Endabschnitt (12b) aufweist, der weiter von dem ersten zylindrischen Abschnitt (11) entfernt ist; und
das zweite Dichtungselement (16) an einer Position angeordnet ist, die näher an dem zweiten proximalen Endabschnitt (12a) liegt als an dem zweiten distalen Endabschnitt (12b).

13. Atemwegadapter (10) nach einem der Ansprüche 9 bis 12, wobei
der zweite zylindrische Abschnitt (12) einen zweiten proximalen Endabschnitt (12a), der näher an dem ersten zylindrischen Abschnitt (11) liegt, sowie einen zweiten distalen Endabschnitt (12b) aufweist, der weiter von dem ersten zylindrischen Abschnitt (11) entfernt ist; und
eine Abmessung des Passabschnitts (121) in der radialen Richtung des zweiten zylindrischen Abschnitts (12) an dem zweiten proximalen Endabschnitt (12a) einen kleineren Wert hat als an dem zweiten distalen Endabschnitt (12b).

14. Atemwegadapter (10) nach einem der Ansprüche 9 bis 13, wobei
das zweite Dichtungselement (16) eine Vielzahl von Dichtungselementen einschließt, die in einer Richtung angeordnet sind, in der das Einsteckelement (30) eingepasst wird.

15. Atemwegadapter (10) nach Anspruch 14, wobei
die zweiten Dichtungselemente (16) aus wenigstens zwei zweiten Dichtungselementen bestehen, die um unterschiedliche Höhen von der Innenumfangsfläche (12c) vorstehen; und
eines der zweiten Dichtungselemente (16), das um eine größere Höhe vorsteht, an einer Position angeordnet ist, an der ein Abstand zwischen einer Außenumfangsfläche des Einsteckelementes (20) und der Innenumfangsfläche (12c) größer ist, wenn das Einsteckelement (30) in den Passabschnitt (20) eingepasst ist.

16. Atemwegadapter (10) nach einem der Ansprüche 9 bis 15, wobei
das zweite Dichtungselement (16) in einer radialen Richtung des ersten zylindrischen Abschnitts (11) von einer Innenumfangsfläche (12c) des zweiten zylindrischen Abschnitts (12) vorsteht und eine geneigte Fläche hat, die sich in einer Richtung erstreckt, in der das Einsteckelement (30) eingepasst wird.

17. Atemwegadapter (10) nach einem der Ansprüche 1 bis 16, der des Weiteren umfasst:
einen Trägerabschnitt (17), der zwischen dem ersten zylindrischen Abschnitt (11) und dem zweiten zylindrischen Abschnitt (12) angeordnet ist und an dem ein Sensor (40) zum Erfassen von Gas in dem Atemweg (131) installiert ist.

## Revendications

1. Adaptateur trachéal (10) formant une partie d'un circuit de respiration raccordé à un sujet de test, l'adaptateur trachéal comprenant :
une première partie cylindrique (11) qui forme une borne mâle ajustée dans un élément femelle (20) formant une autre partie du circuit de respiration ;
une seconde partie cylindrique (12) qui forme une borne femelle compartimentalisant une partie d'ajustement (121) dans laquelle un élément mâle (30) formant une autre partie du circuit de respiration est ajusté ;
une partie formant voie aérienne (13) qui est disposée dans un espace interne (111) compartimentalisé par la première partie cylindrique (11) et qui forme une voie aérienne (131) communiquant avec la partie d'ajustement (121) ; et
un premier élément d'étanchéité (15) qui est disposé sur une surface circonférentielle externe (11d) de la première partie cylindrique (11) et qui présente une flexibilité supérieure à celle de la première partie cylindrique (11).

2. Adaptateur trachéal (10) selon la revendication 1,
le premier élément d'étanchéité (15) ayant une forme d'anneau s'étendant dans un sens circonférentiel de la première partie cylindrique (11).

3. Adaptateur trachéal (10) selon la revendication 1 ou 2,
la première partie cylindrique (11) ayant une première partie d'extrémité proximale (11a) qui est plus proche de la seconde partie cylindrique (12), et une première partie d'extrémité distale (11b) qui est plus éloignée de la seconde partie cylindrique (12) ; et
le premier élément d'étanchéité (15) étant disposé au niveau d'une position plus proche de la première partie d'extrémité distale (11b) que de la première partie d'extrémité proximale (11a).

4. Adaptateur trachéal (10) selon la revendication 1 ou 2,
la première partie cylindrique (11) ayant une première partie d'extrémité proximale (11a) qui est plus proche de la seconde partie cylindrique (12), et une première partie d'extrémité distale (11b) qui est plus éloignée de la seconde partie cylindrique (12) ; et
le premier élément d'étanchéité (15) étant disposé au niveau d'une position plus proche de la première partie d'extrémité proximale (11a) que de la première partie d'extrémité distale (11b).

5. Adaptateur trachéal (10) selon l'une quelconque des revendications 1 à 4,
la première partie cylindrique (11) ayant une première partie d'extrémité proximale (11a) qui est plus proche de la seconde partie cylindrique (12), et une première partie d'extrémité distale (11b) qui est plus éloignée de la seconde partie cylindrique (12) ; et
une dimension dans un sens radial de la première partie cylindrique (11) étant plus petite en valeur au niveau de la première partie d'extrémité distale (11b) qu'au niveau de la première partie d'extrémité proximale (11a).

6. Adaptateur trachéal (10) selon l'une quelconque des revendications 1 à 5,
le premier élément d'étanchéité (15) incluant une pluralité d'éléments d'étanchéité qui sont disposés en rangée le long d'un sens dans lequel la borne mâle est ajustée dans l'élément femelle (20).

7. Adaptateur trachéal (10) selon la revendication 6,
les premiers éléments d'étanchéité (15) étant constitués d'au moins deux premiers éléments d'étanchéité ayant différentes hauteurs de protubérance depuis la surface circonférentielle externe (11d) ; et
l'un des premiers éléments d'étanchéité (15) qui présente une hauteur de protubérance plus grande étant disposé au niveau d'une position où un intervalle entre une surface circonférentielle interne de l'élément femelle (20) et la surface circonférentielle externe (11d) est plus grand lorsque la première partie cylindrique (11) est ajustée dans l'élément femelle (20).

8. Adaptateur trachéal (10) selon l'une quelconque des revendications 1 à 7,
le premier élément d'étanchéité (15) faisant saillie dans un sens radial de la première partie cylindrique (11) depuis la surface circonférentielle externe (11d) de la première partie cylindrique (11), et ayant une surface inclinée le long d'un sens dans lequel la borne mâle est ajustée dans l'élément femelle (20).

9. Adaptateur trachéal (10) selon l'une quelconque des revendications 1 à 8, comprenant en outre :
un second élément d'étanchéité (16) qui est disposé sur une surface circonférentielle interne (12c) de la seconde partie cylindrique (12) dans la partie d'ajustement (121), et présentant une flexibilité supérieure à celle de la seconde partie cylindrique (12).

10. Adaptateur trachéal (10) selon la revendication 9,
le second élément d'étanchéité (16) ayant une forme d'anneau qui s'étend dans un sens circonférentiel de la seconde partie cylindrique (12).

11. Adaptateur trachéal (10) selon la revendication 9 ou 10,
la partie d'ajustement (121) ayant une seconde partie d'extrémité proximale (12a) qui est plus proche de la première partie cylindrique (11), et une seconde partie d'extrémité distale (12b) qui est plus éloignée de la première partie cylindrique (11) ; et
le second élément d'étanchéité (16) étant disposé au niveau d'une position plus proche de la seconde partie d'extrémité distale (12b) que de la seconde partie d'extrémité proximale (12a).

12. Adaptateur trachéal (10) selon la revendication 9 ou 10,
la partie d'ajustement (121) ayant une seconde partie d'extrémité proximale (12a) qui est plus proche de la première partie cylindrique (11), et une seconde partie d'extrémité distale (12b) qui est plus éloignée de la première partie cylindrique (11) ; et
le second élément d'étanchéité (16) étant disposé au niveau d'une position plus proche de la seconde partie d'extrémité proximale (12a) que de la seconde partie d'extrémité distale (12b).

13. Adaptateur trachéal (10) selon l'une quelconque des revendications 9 à 12,
la seconde partie cylindrique (12) ayant une seconde partie d'extrémité proximale (12a) qui est plus proche de la première partie cylindrique (11), et une seconde partie d'extrémité distale (12b) qui est plus éloignée de la première partie cylindrique (11) ; et
une dimension de la partie d'ajustement (121) dans le sens radial de la seconde partie cylindrique (12) étant plus petite en valeur au niveau de la seconde partie d'extrémité proximale (12a) qu'au niveau de la seconde partie d'extrémité distale (12b).

14. Adaptateur trachéal (10) selon l'une quelconque des revendications 9 à 13,
le second élément d'étanchéité (16) incluant une pluralité d'éléments d'étanchéité qui sont disposés en rangée le long d'un sens dans lequel l'élément mâle (30) est ajusté.

15. Adaptateur trachéal (10) selon la revendication 14,
les seconds éléments d'étanchéité (16) étant constitués d'au moins deux seconds éléments d'étanchéité ayant différentes hauteurs de protubérance depuis la surface circonférentielle interne (12c) ; et
l'un des seconds éléments d'étanchéité (16) qui présente une hauteur de protubérance plus grande étant disposé au niveau d'une position où un intervalle entre une surface circonférentielle externe de l'élément mâle (30) et la surface circonférentielle interne (12c) est plus grand lorsque l'élément mâle (30) est ajusté dans la partie d'ajustement (121).

16. Adaptateur trachéal (10) selon l'une quelconque des revendications 9 à 15,
le second élément d'étanchéité (16) faisant saillie dans un sens radial de la première partie cylindrique (11) depuis une surface circonférentielle interne (12c) de la seconde partie cylindrique (12) et présentant une surface inclinée s'étendant le long d'un sens dans lequel l'élément mâle (30) est ajusté.

17. Adaptateur trachéal (10) selon l'une quelconque des revendications 1 à 16, comprenant en outre :
une partie de support (17) qui est disposée entre la première partie cylindrique (11) et la seconde partie cylindrique (12), et sur laquelle un capteur (40) pour détecter du gaz dans la voie aérienne (131) est monté.
